(19) Europäisches Patentamt
European Patent Office
Office européen des brevets

(11) Veröffentlichungsnummer: **0 093 696**
**B1**

(12) # EUROPÄISCHE PATENTSCHRIFT

(45) Veröffentlichungstag der Patentschrift: **04.06.86**

(51) Int. Cl.⁴: **C 07 C 69/738,** C 07 C 59/82, C 07 F 9/40 // C07C69/732

(21) Anmeldenummer: **83810181.4**

(22) Anmeldetag: **29.04.83**

(54) **Benzo-1,4-chinone.**

(30) Priorität: **05.05.82 GB 8212970**

(43) Veröffentlichungstag der Anmeldung: **09.11.83 Patentblatt 83/45**

(45) Bekanntmachung des Hinweises auf die Patenterteilung: **04.06.86 Patentblatt 86/23**

(84) Benannte Vertragsstaaten: **BE DE FR GB IT**

(56) Entgegenhaltungen:
**EP - A - 0 021 841**
**US - A - 3 957 836**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Howell, Frederick Harold, Dr., 27 High Bank, Atherton Lancashire M29 9HZ (GB)**

**EP 0 093 696 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Benzo-1,4-chinone und ein Verfahren zu deren Herstellung.

Benzochinone mit funktionellen Gruppen sind beispielsweise in der EP-Patentanmeldung Nr. 38092 beschrieben.

Die neuen erfindungsgemässen Benzo-1,4-chinone entsprechen der Formel I

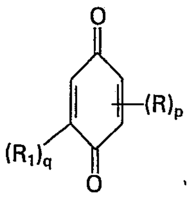
(I),

worin p 1 oder 2 und q 0 oder 1 sind, mit der Massgabe, dass p+q 1 oder 2 ist,

R einen Rest der Formel II

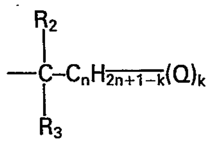
(II),

und

Q $-COOR_4$, $-CON(R)_4)(R_5)$, $-OR_5$, $-OCOR_7$, $-N(R_8)(R_9)$, $-PO(OR_{10})([O]_xR_{11})$, $-SO_2R_{12}$, $-CN$, Halogen, $-NO_2$ oder $-COR_{13}$ bedeuten,

n eine ganze Zahl von 1 bis 20,

k die Zahl 1 oder 2

x die Zahl 0 oder 1 sind,

$R_1$ $C_{1-8}$-Alkyl oder ein Rest der Formel II ist, wobei durch R und $R_1$ dargestellte Reste der Formel II gleich oder verschieden sein können, und

$R_2$ und $R_3$ unabhängig voneinander $C_{1-5}$-Alkyl sind, wobei, wenn Q $-COOR_4$ darstellt, eines von $R_2$ und $R_3$ durch eine Gruppe $-COOR_4$ substituiert sein kann, oder $R_2$ oder $R_3$ so mit dem Rest $-C_nH_{2n+1-k}$ verbunden sein können, dass ein durch die Gruppe (n) $-(COOR_4)_k$ mit gleichen oder verschiedenen $R_4$ substituierter $C_{5-12}$-Cycloalkylenrest gebildet wird,

$R_4$ Wasserstoff, $C_{1-20}$-Alkyl, das durch 1–5 Sauerstoffatome unterbrochen und durch eine Gruppe $-OR_6$ substituiert sein kann, $C_{3-20}$-Alkenyl, $C_{3-12}$-Cycloalkyl, $C_{6-20}$-Aryl, das durch eine $C_{1-4}$-Alkylgruppe substituiert sein kann, $C_{7-13}$-Aralkyl, einen 5- oder 6-gliedrigen, Sauerstoffhaltigen Heterocyclus, der durch eine oder zwei $C_{1-4}$-Alkylgruppen substituiert sein kann, oder Methyl darstellt, das durch einen 5- oder 6-gliedrigen, ein Sauerstoffatom enthaltenden, unsubstituierten oder durch eine oder zwei $C_{1-4}$-Alkylgruppen substituierten Heterocyclus substituiert ist,

$R_5$ Wasserstoff oder $C_{1-20}$-Alkyl bedeutet oder $R_4$ und $R_5$ zusammen mit dem Stickstoffatom, an das sie gebunden sind, einen 5- oder 6-gliedrigen Heterocyclus bilden, der durch eine oder zwei $C_{1-4}$-Alkylgruppen substituiert sein kann,

$R_6$ $C_{3-12}$-Cycloalkyl, $C_{3-20}$-Alkenyl, $C_{6-10}$-Aryl, das durch eine oder zwei $C_{1-4}$-Alkylgruppen substituiert sein kann, oder $C_{7-13}$-Aralkyl bedeutet, und

$R_7$ Wasserstoff, $C_{1-20}$-Alkyl, $C_{3-20}$-Alkenyl, $C_{3-12}$-Cycloalkyl, $C_{7-13}$-Aralkyl oder $C_{6-10}$-Aryl darstellt, das durch eine oder zwei $C_{1-4}$-Alkylgruppen substituiert sein kann,

$R_8$ Wasserstoff oder $C_{1-4}$-Alkyl und

$R_9$ Wasserstoff, $C_{1-4}$-Alkyl oder eine Gruppe $-COR_7$ bedeuten, wobei $R_7$ die oben angegebene Bedeutung hat, oder $R_8$ und $R_9$ zusammen mit dem Stickstoffatom, an das sie gebunden sind, einen substituierten oder durch eine oder zwei $C_{1-4}$-Alkylgruppen substituierten 5- oder 6-gliedrigen Ring bilden, und

$R_{10}$ und $R_{11}$ bei x=1 unabhängig voneinander Wasserstoff, $C_{1-20}$-Alkyl oder zusammen $C_{2-3}$-Alkylen darstellen, das durch 1–4 $C_{1-20}$-Alkylgruppen substituiert sein kann, und bei x=0 $R_{10}$ Wasserstoff oder $C_{1-20}$-Alkyl und $R_{11}$ geradkettiges $C_{1-5}$-Alkyl bedeuten,

$R_{12}$ $-OH$, $-Cl$ oder $-N(R_5)(R_7)$ bedeutet, und

$R_{13}$ $-H$, $C_{1-20}$-Alkyl oder Halogen bedeutet, wobei $R_5$ und $R_7$ die oben angegebene Bedeutung haben, mit der Massgabe, dass $R_1$ einen Rest der Formel II darstellt, wenn $R_{12}$–OH ist,

und Salze von Verbindungen der Formel I mit organischen oder anorganischen Säuren und Basen.

Durch $R_1$ bis $R_{11}$ und $R_{13}$ dargestellte Alkyl- oder Alkenylgruppen sowie Alkylsubstituenten an Resten $R_4$, $R_4$ und $R_5$, $R_6$, $R_7$, $R_8$ und $R_9$, $R_{10}$ und $R_{11}$ können, sofern nichts anderes angegeben ist, geradkettig oder verzweigt sein.

Ist $R_1$ geradkettiges oder verzweigtes $C_{1-8}$-Alkyl, so handelt es sich z.B. um Methyl, Äthyl, n-Propyl, Isopropyl, n-Butyl, Isobutyl, tert.-Butyl, tert.-Pentyl oder 1,1,3,3-Tetramethylbutyl. Beispiele von geradkettigen oder verzweigten $C_{1-4}$-Alkylgruppen $R_2$ und $R_3$ sind die Methyl-, Äthyl-, n-Propyl-, Isopropyl-, n-Butyl- und sek.-Butylgruppen. $C_5$-Alkyl als $R_2$ oder $R_3$ sind beispielsweise n-Pentyl- und Neopentylgruppen. Bedeutet $R_4$ geradkettiges oder verzweigtes $C_{1-20}$-Alkyl, das durch 1–5 Sauerstoffatome unterbrochen sein kann, so kommen z.B. die folgenden Gruppen in Betracht: Methyl, Äthyl, 2-Methoxyäthyl, 2-Äthoxyäthyl, 2-n-Propoxyäthyl, 2-n-Butoxyäthyl, n-Pentyl, n-Hexyl, n-Heptyl, n-Octyl, 2-Äthylhexyl, n-Nonyl, n-Decyl, n-Dodecyl, n-Tetradecyl, n-Hexadecyl, n-Octadecyl, n-Eikosyl, $-(C_2H_4O)_2CH_3$, $-(C_2H_4O)_3CH_3$, $-(C_2H_4O)_4CH_3$ oder $-(C_2H_4O)_5CH_3$. Beispiele von geradkettigen oder verzweigten Alkylengruppen $R_4$ sind $-CH_2CH_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_{10}-$, $-(CH_2)_{12}-$, $-(CH_2)_{16}-$, $-(CH_2)_{18}-$, und $-(CH_2)_{20}-$. Beispiele von geradkettigen oder verzweigten Alkenylgruppen $R_4$, $R_6$ oder $R_7$ sind: Prop-2-enyl, n-But-2-enyl, 2-Methyl-prop-2-enyl, n-Pent-2-enyl, n-Hex-2-enyl, n-Hexa-2,4-dienyl, n-Dec-10-enyl oder n-Eikos-2-enyl.

Stellen $R_4$, $R_6$ oder $R_7$ $C_{3-12}$-Cycloalkylgruppen dar, so kommen z.B. Cyclopropyl-, Cyclobutyl-, Cyclopentyl-, Cyclohexyl-, Cyclooctyl-, Cyclodecyl-, Adamantyl- oder Cyclododecylgruppen in Betracht. Als Beispiele von $C_{7-13}$-Aralkylgruppen $R_4$,

$R_6$ oder $R_7$ seien Benzyl, Phenyläthyl, Benzhydryl und Naphthylmethyl genannt. Bedeuten $R_4$, $R_6$ oder $R_7$ $C_{6-10}$-Aryl, das durch eine oder zwei $C_{1-4}$-Alkylgruppen substitutiert sein kann, so handelt es sich z. B. um Phenyl-, Tolyl-, Xylyl-, Cumyl-, Butylphenyl- oder Naphthylgruppen.

Stellt $R_4$ einen 5- oder 6-gliedrigen, Sauerstoffhaltigen Heterocyclus dar, der durch eine oder zwei $C_{1-4}$-Alkylgruppen substituiert sein kann, so handelt es sich z. B. um Tetrahydrofuran-3-yl, Tetrahydropyran-4-yl oder 2,6-Dimethyl-tetrahydropyran-4-yl. Ist $R_4$ Methyl, das durch einen 5- oder 6-gliedrigen, ein Sauerstoffatom enthaltenden und gegebenenfalls durch eine oder zwei $C_{1-4}$-Alkylgruppen substituierten Heterocyclus substituiert ist, so kommen beispielsweise Furfuryl, Tetrahydrofurfuryl oder Tetrahydropyran-2-yl-methyl in Betracht.

Bedeuten $R_8$ oder $R_9$ geradkettiges oder verzweigtes $C_{1-4}$-Alkyl, so handelt es sich z. B. um Methyl, Äthyl, n-Propyl, Isopropyl, n-Butyl, oder sek.-Butyl. Bilden $R_4$ und $R_5$ oder $R_8$ und $R_9$ zusammen mit dem Stickstoffatom, an das sie gebunden sind, einen 5- oder 6-gliedrigen Heterocyclus, der durch eine oder zwei $C_{1-4}$-Alkylgruppen substituiert sein kann, so kann es sich z. B. um einen Pyrrolidin-, Piperidin-, Morpholin- oder 2,5-Dimethylmorpholinring handeln.

Geradkettige oder verzweigte $C_{1-20}$-Alkylgruppen $R_5$, $R_7$, $R_{10}$, $R_{11}$ oder $R_{13}$ können gleich oder verschieden sein und z. B. Methyl, Äthyl, n-Propyl, Isopropyl, n-Butyl, sek-Butyl, n-Pentyl, n-Hexyl, n-Heptyl, n-Octyl, 2-Äthylhexyl, n-Nonyl, n-Decyl, n-Dodecyl, n-Tetradecyl, n-Hexadecyl, n-Octadecyl oder n-Eikosyl darstellen.

Bilden $R_{10}$ und $R_{11}$ zusammen gegebenenfalls durch 1–4 $C_{1-20}$-Alkylgruppen substituiertes $C_{2-3}$-Alkylen, so kommen z. B. die folgenden Gruppen in Betracht: $-CH_2CH_2-$, $-CH_2CH_2CH_2-$, $-CH_2CH(CH_3)-$, $-CH_2CH(C_2H_5)-$, $-CH_2CH(C_{20}H_{41})-$, $-CH(CH_3)CH(CH_3)-$, $-CH(CH_3)C(CH_3)_2-$, $-C(CH_3)_2C(CH_3)_2-$, $-CH_2CH_2C(CH_3)_2-$ oder $-CH(CH_3)CH_2CH(CH_3)-$.

Als Beispiele von Salzen, wenn Q eine saure Gruppe, d. h. –COOH darstellt, seien Salze mit Alkali- und Erdalkalimetallen und Aminen genannt. Ist Q eine Gruppe $-N(R_8)(R_9)$, so kommen Salze mit organischen oder anorganischen Säuren, z. B. Chlorwasserstoffsäure, Schwefelsäure, para-Toluolsulfonsäure und Oxalsäure, in Betracht.

Gemäss einer bevorzugten Ausführungsform sind R und $R_1$ in 2- und 5-Stellung an das Benzo-1,4-chinon der Formel I gebunden. Des weiteren bevorzugt sind Verbindungen der Formel I, worin $R_1$ einen Rest der Formel III

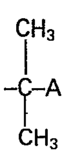

(III)

bedeutet, worin A Alkyl mit 1 bis 5 Kohlenstoffatomen ist.

Vorzugsweise ist Q in dem Substituenten R der Verbindung der Formel I $-COOR_4$, $-OCOR_7$ oder –CN, wobei $R_4$ und $R_7$ die angegebenen Bedeutungen haben.

In besonders bevorzugten Verbindungen der Formel I ist R ein Rest der Formel II, worin Q $-COOR_4$ ist, wobei $R_4$ $C_{1-12}$-Alkyl, das unsubstituiert oder durch $C_{3-12}$-Cycloalkyloxy oder $C_{6-10}$-Aryloxy substituiert ist, $C_{3-12}$-Cycloalkyl, $C_{6-10}$-Aryl, $C_{7-13}$-Aralkyl oder Methyl darstellt, das durch einen 5- oder 6-gliedrigen, ein Sauerstoffatom enthaltenden, unsubstituierten heterocyclischen Ring substituiert ist, oder Q $-OCOR_7$ ist, worin $R_7$ Wasserstoff, $C_{1-20}$-Alkyl, $C_{3-12}$-Cycloalkyl, $C_{7-13}$-Aralkyl oder unsubstituiertes $C_{6-10}$-Aryl bedeutet, $R_1$ ein Rest der Formel II ist, worin Q die angegebene Bedeutung hat, $R_2$ und $R_3$ unabhängig voneinander Alkyl mit 1 bis 5 Kohlenstoffatomen sind, p und q 1 sind und k und n die oben angegebenen Bedeutungen haben.

Eine weitere Gruppe besonders geeigneter Verbindungen der Formel I entspricht der Formel IV

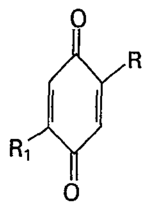

(IV),

in welcher R und $R_1$ ein Rest der Formel II sind, worin Q $-COOR_4$, wobei $R_4$ Alkyl mit 1 bis 8 Kohlenstoffatomen oder Cycloalkyl mit 5 bis 8 Kohlenstoffatomen ist, oder $-OCOR_7$ bedeutet, worin $R_7$ Wasserstoff oder Alkyl mit 1 bis 4 Kohlenstoffatomen ist, $R_2$ und $R_3$ jeweils Methyl, k 1 und n 1 bis 10 sind.

Ebenfalls besonders bevorzugt sind Verbindungen der Formel IV, worin R und $R_1$ ein Rest der Formel II sind, Q $-COOR_4$, $R_2$ und $R_3$ Methyl, $R_4$ $C_{1-8}$-Alkyl, k die Zahl 1 und n die Zahl 3 sind.

Als Beispiele von Verbindungen der Formel I seien genannt:

2-(3'-Methoxycarbonyl-2'-methyl-prop-2'-yl)-1,4-benzochinon,
2-(3'-n-Hexyloxycarbonyl-2'-methyl-prop-2'-yl)-1,4-benzochinon,
5-t-Butyl-2-(3'-n-hexyloxycarbonyl-2'-methyl-prop-2'-yl)-1,4-benzochinon,
5-t-Butyl-2-(3'-n-dodecyloxycarbonyl-2'-methyl-prop-2'-yl)-1,4-benzochinon,
2,5-Bis-(3'-methoxycarbonyl-2'-methyl-prop-2'-yl)-1,4-benzochinon,
2,5-Bis-(3'-n-hexyloxycarbonyl-2'-methyl-prop-2'-yl)-1,4-benzochinon,
2,5-Bis-(3'-n-dodecyloxycarbonyl-2'-methyl-prop-2'-yl)-1,4-benzochinon,
2-(4'-Methoxycarbonyl-1'-methyl-cyclohex-1'-yl)-1,4-benzochinon,
2-(4'-Hexyloxycarbonyl-1'-methyl-cyclohex-1'-yl)-1,4-benzochinon,
2,5-Bis-(4'-methoxycarbonyl-1'-methyl-cyclohex-1'-yl)-1,4-benzochinon,
2,5-Bis-(4'-n-hexyloxycarbonyl-1'-methyl-cyclohex-1'-yl)-1,4-benzochinon,

2-(5′-Methoxycarbonyl-2′-methyl-pent-2′-yl)-1,4-benzochinon,
2-(5′-n-Hexyloxycarbonyl-2′-methyl-pent-2′-yl)-1,4-benzochinon,
2-(5′-Hexyloxycarbonyl-2′-methyl-pent-2′-yl)-5-methyl-1,4-benzochinon,
2-(5′-n-Hexyloxycarbonyl-2′-methyl-pent-2′-yl)-6-methyl-1,4-benzochinon,
2-(5′-Carboxy-2′-methyl-pent-2′-yl)-5-t-butyl-1,4-benzochinon,
2-(5′-Methoxycarbonyl-2′-methyl-pent-2′-yl)-5-t-butyl-1,4-benzochinon,
2-(5′-n-Hexyloxycarbonyl-2′-methyl-pent-2′-yl)-5-t-butyl-1,4-benzochinon,
2-(5′-n-Dodecyloxycarbonyl-2′-methyl-pent-2′-yl)-5-t-butyl-1,4-benzochinon,
2-(5′-Methoxycarbonyl-2′-methyl-pent-2′-yl)-5-(1′, 1′, 3′, 3′-tetramethylbutyl)-1,4-benzochinon,
2,5-Bis-(5′-methoxycarbonyl-2′-methyl-pent-2′-yl)-1,4-benzochinon,
2,5-Bis-(5′-äthoxycarbonyl-2′-methyl-pent-2′-yl)-1,4-benzochinon,
2,5-Bis-(5′-n-propyloxycarbonyl-2′-methyl-pent-2′-yl)-1,4-benzochinon,
2-(5′-n-Hexyloxycarbonyl-2′-methyl-pent-2′-yl)-5-(3′-n-hexyloxycarbonyl-2′-methyl-prop-2′-yl)-1,4-benzochinon,
2-(5′-n-Hexyloxycarbonyl-2′-methyl-pent-2′-yl)-5-(3′-n-octyloxycarbonyl-2′-methyl-prop-2′-yl)-1,4-benzochinon,
2,5-Bis-(5′-iso-propyloxycarbonyl-2′-methyl-pent-2′-yl)-1,4-benzochinon,
2,5-Bis-(5′-n-butyloxycarbonyl-2′-methyl-pent-2′-yl)-1,4-benzochinon,
2,5-Bis-(5′-iso-butyloxycarbonyl-2′-methyl-pent-2′-yl)-1,4-benzochinon,
2,5-Bis-(5′-n-pentyloxycarbonyl-2′-methyl-pent-2′-yl)-1,4-benzochinon,
2,5-Bis-(5′-iso-pentyloxycarbonyl-2′-methyl-pent-2′-yl)-1,4-benzochinon,
2,5-Bis-(5′-n-hexyloxycarbonyl-2′-methyl-pent-2′-yl)-1,4-benzochinon,
2,5-Bis-(5′-n-heptyloxycarbonyl-2′-methyl-pent-2′-yl)-1,4-benzochinon,
2,5-Bis-(5′-cyclohexylocycarbonyl-2′-methyl-pent-2′-yl)-1,4-benzochinon,
2,5-Bis-(5′-n-octyloxycarbonyl-2′-methyl-pent-2′-yl)-1,4-benzochinon,
2,5-Bis-[5′-(2″-äthylhexyloxycarbonyl)-2′-methyl-pent-2′-yl]-1,4-benzochinon,
2,5-Bis-(5′-n-dodecyloxycarbonyl-2′-methyl-pent-2′-yl)-1,4-benzochinon,
2,5-Bis-(5′-n-hexadecyloxycarbonyl-2′-methyl-pent-2′-yl)-1,4-benzochinon,
2,5-Bis-[5′-(2″-methoxyäthoxycarbonyl)-2′-methyl-pent-2′-yl]-1,4-benzochinon,
2,5-Bis-[5′-(2″-n-butyloxyäthoxycarbonyl)-2′-methyl-pent-2′-yl]-1,4-benzochinon,
2,5-Bis-[5′-(2″-cyclohexyloxyäthoxycarbonyl)-2′-methyl-pent-2′-yl]-1,4-benzochinon,
2,5-Bis-[5′-(2″-allyloxyäthoxycarbonyl)-2′-methyl-pent-2′-yl]-1,4-benzochinon,
2,5-Bis-[5′-(2″-benzyloxyäthoxycarbonyl)-2′-methyl-pent-2′-yl]-1,4-benzochinon,
2,5-Bis-[5′-(2″-phenoxyäthoxycarbonyl)-2′-methyl-pent-2′-yl]-1,4-benzochinon,
2,5-Bis-(5′-phenoxycarbonyl-2′-methyl-pent-2′-yl)-1,4-benzochinon,
2,5-Bis-(5′-benzyloxycarbonyl-2′-methyl-pent-2′-yl)-1,4-benzochinon,
2,5-Bis-(5′-tetrahydrofurfuryloxycarbonyl-2′-methyl-pent-2′-yl)-1,4-benzochinon,
2,5-Bis-(5′-furfuryloxycarbonyl-2′-methyl-pent-2′-yl)-1,4-benzochinon,
2,5-Bis-(5′-tetrahydropyran-4″-yloxycarbonyl-2′-methyl-pent-2′-yl)-1,4-benzochinon,
2-(5′-n-Hexyloxycarbonyl-2′-methyl-pent-2′-yl)-5-(5′-methoxycarbonyl-2′-methyl-pent-2′-yl)-1,4-benzochinon,
2,5-Bis-(5′-carboxy-2′-methyl-pent-2′-yl)-1,4-benzochinon und dessen Na-Salze,
2,5-Bis-(5′-carbamoyl-2′-methyl-pent-2′-yl)-1,4-benzochinon,
2,5-Bis-(5′-N-n-butylcarbamoyl-2′-methyl-pent-2′-yl)-1,4-benzochinon,
2,5-Bis-(5′-N-n-dimethylcarbamoyl-2′-methyl-pent-2′-yl)-1,4-benzochinon,
2,5-Bis-(5′-N,N-di-n-butylcarbamoyl-2′-methyl-pent-2′-yl)-1,4-benzochinon,
2,5-Bis-(5′-N-eikosylcarbamoyl-2′-methyl-pent-2′-yl)-1,4-benzochinon,
2,5-Bis-(5′-N-allylcarbamoyl-2′-methyl-pent-2′-yl)-1,4-benzochinon,
2,5-Bis-(5′-N-cyclohexylcarbamoyl-2′-methyl-pent-2′-yl)-1,4-benzochinon,
2,5-Bis-(5′-N-phenylcarbamoyl-2′-methyl-pent-2′-yl)-1,4-benzochinon,
2,5-Bis-(5′-N-benzylcarbamoyl-2′-methyl-pent-2′-yl)-1,4-benzochinon,
2,5-Bis-(5′-morpholinocarbamoyl-2′-methyl-pent-2′-yl)-1,4-benzochinon,
2-(7′-Methoxycarbonyl-2′,2′,4′-trimethyl-hept-4′-yl)-5-t-butyl-1,4-benzochinon,
2-(1′,7′-Di-methoxycarbonyl-4′-methyl-hept-4′-yl)-1,4-benzochinon,
2,5-Bis-(2′,6′-dimethyl-8′-hydroxy-oct-2′-yl)-1,4-benzochinon,
2,5-Bis-(8′-acetyloxy-2′,6′-dimethyl-oct-2′-yl)-1,4-benzochinon,
2,5-Bis-(2′,6′-dimethyl-8′-propionyloxy-oct-2′-yl)-1,4-benzochinon,
2,5-Bis-(8′-butyryloxy-2′,6′-dimethyl-oct-2′-yl)-1,4-benzochinon,
2,5-Bis-(2′,6′-dimethyl-8′-hexanoyloxy-2′-yl)-1,4-benzochinon,
2-(5′-Diäthylphosphono-5′-äthoxycarbonyl-2′-methyl-pent-2′-yl)-1,4-benzochinon,
2,5-Bis-(5′,5′-di-äthoxycarbonyl-2′-methyl-pent-2′-yl)-1,4-benzochinon,
2,5-Bis-(2′,6′-dimethyl-8′-eikosyloxy-oct-2′-yl)-1,4-benzochinon,
2,5-Bis-(8′-crotonyloxy-2′,6′-dimethyl-oct-2′-yl)-1,4-benzochinon,
2,5-Bis-(8′-benzoyloxy-2′,6′-dimethyl-oct-2′-yl)-1,4-benzochinon,
2,5-Bis-(2′,6′-dimethyl-8′-phenacetyloxy-oct-2′-yl)-1,4-benzochinon,
2,5-Bis-(8′-cyclohexylcarbonyloxy-2′,6′-dimethyl-oct-2′-yl)-1,4-benzochinon,
2,5-Bis-(8′-methoxy-2′,6′-dimethyl-oct-2′-yl)-1,4-benzochinon,
2,5-Bis-(8′-n-butoxy-2′,6′-dimethyl-oct-2′-yl)-1,4-benzochinon,
2,5-Bis-(6′-amino-2′-methyl-hept-2′-yl)-1,4-benzochinon und dessen Hydrochlorid,
2,5-Bis-(6′-N-methylamino-2′-methyl-hept-2′-yl)-1,4-benzochinon,
2,5-Bis-(6′-N,N-dimethylamino-2′-methyl-hept-2′-yl)-1,4-benzochinon,
2,5-Bis-(6′-N-äthylamino-2′-methyl-hept-2′-yl)-1,4-benzochinon,
2,5-Bis-(6′-N,N-diäthylamino-2′-methyl-hept-2′-yl)-1,4-benzochinon,
2,5-Bis-(6′-N-n-butylamino-2′-methyl-hept-2′-yl)-1,4-benzochinon,
2,5-Bis-(6′-N,N-di-n-butylamino-2′-methyl-hept-2′-yl)-1,4-benzochinon,
2,5-Bis-(2′-methyl-6′-morpholino-hept-2′-yl)-1,4-benzochinon,
2,5-Bis-(6′-acetamido-2′-methyl-hept-2′-yl)-1,4-benzochinon,
2,5-Bis-(6′-hexanamido-2′-methyl-hept-2′-yl)-1,4-benzochinon,
2,5-Bis-(12′-amino-2′,13′-dimethyl-tetradec-2′-yl)-1,4-benzochinon,
2,5-Bis-(12′-amino-3′,13′-dimethyl-tetradec-3′-yl)-1,4-benzochinon,
2,5-Bis-(12′-acetamido-2′,13′-dimethyl-tetradec-2′-yl)-1,4-benzochinon,
2,5-Bis-(12′-acetamido-3′,13′-dimethyl-tetradec-3′-yl)-1,4-benzochinon,
2-(2′-Methyl-4′-phosphono-but-2′-yl)-1,4-benzochinon und dessen Natriumsalze.
2-(2′-Methyl-4′-dimethylphosphono-but-2′-yl)-1,4-benzochinon,
2-(4′-Diäthylphosphono-2′-methyl-but-2′-yl)-1,4-benzochinon,
2-(4′-Di-n-butylphosphono-2′-methyl-but-2′-yl)-1,4-benzochinon,
5-t-Butyl-2-(2′-methyl-4′-dimethylphosphono-but-2′-yl)-1,4-benzochinon,
5-(1′,1′,3′,3′-Tetramethylbutyl)-2-(2′-methyl-4′-dimethylphosphonobut-2′-yl)-1,4-benzochinon,
2,5-Bis-(2′-methyl-4′-di-methylphosphono-but-2′-yl)-1,4-benzochinon,
2,5-Bis-(2′-methyl-4′-di-äthylphosphono-but-2′-yl)-1,4-benzochinon,
2,5-Bis-(2′-methyl-4′-di-n-propylphosphono-but-2′-yl)-1,4-benzochinon,
2-(5′-n-Hexyloxycarbonyl-2′-methyl-pent-2′-yl)-5-(2′-methyl-4′-diäthylphosphono-but-2′-yl)-1,4-benzochinon,
2,5-Bis-(2′-methyl-4′-di-iso-propylphosphono-but-2′-yl)-1,4-benzochinon,
2,5-Bis-(2′-methyl-4′-di-n-butyl-phosphono-but-2′-yl)-1,4-benzochinon,
2,5-Bis-[2′-methyl-4′-(di-2″-äthylhexyl-phosphono)-but-2′-yl]-1,4-benzochinon,
2,5-Bis-(2′-methyl-4′-di-n-dodecylphosphono-but-2′-yl)-1,4-benzochinon,
2,5-Bis-[2′-methyl-4′-(2-oxo-1,3,2-dioxaphospholan-2″-yl)-but-2′-yl]-1,4-benzochinon,
2,5-Bis-[2′-methyl-4′-(4-methyl-2-oxo-1,3,2-dioxaphospholan-2″-yl)-but-2′-yl]-1,4-benzochinon,
2,5-Bis-[4′-(äthyl-äthylphosphino)-2′-methyl-but-2′-yl]-1,4-benzochinon,
2,5-Bis-(2′-methyl-3′-sulfo-prop-2′-yl)-1,4-benzochinon,
2-(2′-Methyl-3′-sulfonamido-prop-2′-yl)-1,4-benzochinon,
5-t-Butyl-2-(2′-methyl-3′-sulfonamido-prop-2′-yl)-1,4-benzochinon,
2-(2′-Methyl-3′-N-methylsulfonamido-prop-2′-yl)-1,4-benzochinon,
5-(1′,1′,3′,3′-Tetramethylbutyl)-2-(2′-methyl-3′-N-methylsulfonamido-prop-2′-yl)-1,4-benzochinon,
2-(2′-Methyl-3′-N,N-di-n-butylsulfonamido-prop-2′-yl)-1,4-benzochinon,
2-(2′-Methyl-3′-N-n-octylsulfonamido-prop-2′-yl)-1,4-benzochinon,
5-t-Butyl-2-(2′-methyl-3′-N-n-octylsulfonamido-prop-2′-yl)-1,4-benzochinon,
5-t-Butyl-2-(2′-methyl-5′-äthyloxycarbonyl-5′-diäthylphosphono-hex-2′-yl)-1,4-benzochinon,
2,5-Bis-(2′,6′-dimethyl-7′-carboxy-hept-2′-yl)-1,4-benzochinon,
2,5-Bis-(2′,6′-dimethyl-7′-methyloxycarbonyl-hept-2′-yl)-1,4-benzochinon.

Die Verbindungen der Formel I können durch Oxidation einer Verbindung der Formel V

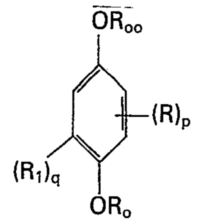 (V),

worin R, $R_1$, p und q die unter Formel I angegebene Bedeutung haben, und $R_o$ und $R_{oo}$ unabhängig voneinander –H oder $-CH_3$ bedeuten, hergestellt werden.

Geeignete Oxidationsmittel sind z.B. Salze von elektronenanziehenden Verbindungen mit einem geeigneten Redoxpotential, wie Eisen(III)-, Kupfer(II)-, Quecksilber(II)- und Cerium(IV)-salze, Silberoxid, Silbercarbonat, Bleitetraacetat, Chromsäure und Chromate, Chromoxid, beispielsweise Jones Reagenz, Mangandioxid und Permanganate, Hypohalogenite, z.B. Natriumhypochlorit; Perhalogenate, z.B. Natriumperchlorat allein oder mit Metavanadatsalzen katalysiert; Salpetersäure, Nitrate und Oxide des Stickstoffs; Sauerstoff und Luft, gegebenenfalls katalysiert durch Metallsalze, wie Kupfersalze. Es können auch organische Oxidationsmittel verwendet werden, z.B. Chloranil, sowie organometallische oder organisch komplexierte anorganische Oxidationsmittel. Bevorzugt verwendet man als Oxidationsmittel Hypohalogenit-Salze, Sauerstoff oder Luft in Gegenwart von Kupfersalzen. Für die Umsetzung wird mindestens ein Äquivalent Oxidationsmittel und üblicherweise ein Überschuss an Oxidationsmittel eingesetzt. Die Umsetzung kann in wässriger oder organischer Lösung oder Suspension bei Temperaturen zwischen 0°C und dem Siedepunkt des Lösungs- bzw. Suspensionsmittels, bevorzugt bei 0–40°C, durchgeführt werden. Die Reaktion kann auch in einem Zweiphasensystem in Gegenwart eines Phasentransfer-Katalysators nach dem in der britischen Patentpublikation 2.070.003 beschriebenen Verfahren vorgenommen werden.

Als organische Lösungsmittel kommen solche in Betracht, die unter den Reaktionsbedingungen stabil sind, z.B. Chloroform, Methylenchlorid, Aceton, Benzol, Toluol und Petroläther.

Funktionelle Derivate von Verbindungen der Formel I können auch in andere funktionelle Derivate übergeführt werden. So können z.B. Verbindungen, worin Q –COOH darstellt, mit Alkoholen $R_4OH$ zu den entsprechenden Estern $-COOR_4$ umgesetzt werden. Verbindungen der Formel I, worin Q $-COOR_4$ darstellt, können durch Umesterung in Verbindungen der Formel I mit anderer Bedeutung von $R_4$ übergeführt werden, oder Estergruppen $-COOR_4$ können durch Behandlung mit Verbindungen $NH(R_4)(R_5)$, worin $R_4$ und $R_5$ die oben angegebene Bedeutung haben, in Amide $-CON(R_4)(R_5)$ umgewandelt werden. Wenn Q eine Hydroxylgruppe darstellt, welche an eine Methylengruppe gebunden ist (beispielsweise wie $-CH_2OH$), kann diese Gruppe durch Behandlung beispielsweise mit Chromtrioxid zur Carboxylgruppe oxidiert werden.

Die Ausgangsprodukte der Formel V können dadurch hergestellt werden, dass man bekannte Hydrochinone der Formel VI

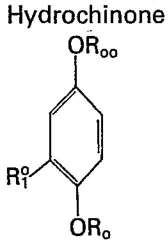

(VI),

worin $R_1^o$ Wasserstoff darstellt oder $R_1$ ist, wobei $R_1$, $R_o$ und $R_{oo}$ die oben angegebene Bedeutung haben, mit einem zur Einführung von Gruppen der Formel II befähigten funktionellen Alkylierungsmittel umsetzt. Als Verbindungen der Formel V können z.B. die den vorstehend erwähnten Benzo-1,4-chinonen entsprechenden Hydrochinone eingesetzt werden. Beispiele für derartige Verbindungen der Formel V, worin $R_o$ und $R_{oo}$ Wasserstoff sind, sind in der EP-Patentanmeldung 69068 beschrieben.

Weitere Beispiele für Verbindungen der Formel V sind:

2-(5′-Carboxy-2′-methyl-pent-2′-yl)-5-t-butyl-hydrochinon

2,5-Bis-(8′-n-butoxy-2′,6′-dimethyl-oct-2′-yl)-hydrochinon

5-t-Butyl-2-(2′-methyl-5′-äthyloxycarbonyl-5′-diäthylphosphono-hex-2′-yl)-hydrochinon

2-(5′-Methoxycarbonyl-2′-methyl-pent-2′-yl)-4-methoxyphenol

3-(5′-Methoxycarbonyl-2′-methyl-pent-2′-yl)-4-methoxyphenol

2-(5′-Methoxycarbonyl-2′-methyl-pent-2′-yl)-1,4-dimethoxybenzol

2,5-Bis-(5′-methoxycarbonyl-2′-methyl-pent-2′-yl)-1,4-dimethoxybenzol.

Zur Umsetzung mit den Hydrochinonen der Formel VI geeignete funktionelle Alkylierungsmittel enthalten ein reaktives Zentrum, z.B. eine olefinische Gruppe oder eine OH-Gruppe, die im Verlaufe der Alkylierungsreaktion eliminiert, umgewandelt oder umgelagert wird.

Geeignete funktionelle Olefine zur funktionellen Alkylierung von Verbindungen der Formel VI sind z.B. 5-Methylhex-5-en-säure, 5-Methyl-hex-5-ensäuremethyl, -äthyl-, -n-propyl-, -isopropyl-, -n-butyl-, -sek-butyl-, -n-pentyl-, -isopentyl-, -sek-pentyl-, -n-hexyl-, -cyclohexyl-, -2′-äthylhexyl-, -n-octyl-, -n-dodecyl- und -n-hexadecylester, 5,7,7-Trimethyl-oct-4-ensäuremethylester, 1,7-Dimethoxycarbonyl-4-methyl-hept-3-en, 4-Methoxycarbonyl-1-methylcyclo-hex-1-en, Dimethylprenylphosphonat, Diäthylprenylphosphonat, Di-n-propylprenylphosphonat, Di-isopropylprenylphosphonat, Di-n-butylprenylphosphonat, Di-n-octylprenylphosphonat, Citronellol, Citronellylacetat, Citronellylmethyläther, Citronellyl-n-butyläther, 2-Amino-6-methyl-hept-5-en, 2-Amino-6-methyl-hept-6-en, 2-Äthoxycarbonyl-5-methyl-hex-4-en-2-phosphonsäurediäthylester, 2-Äthoxycarbonyl-5-methyl-hex-4-ensäureäthylester, 2-Acetamido-6-methyl-hept-5-en, 2-Acetamido-6-methyl-hept-6-en, 2-Methyl-2-propen-1-sulfonsäure, 2-Methyl-2-propen-1-sulfonsäureamid, N-n-Butyl-2-propen-1-sulfonsäureamid, N,N-n-Dibutyl-2-propensulfonsäureamid, N-n-Octyl-2-propen-1-sulfonsäureamid.

Als Beispiele für funktionelle Hydroxyverbindungen zur Alkylierung von Verbindungen der Formel VI seien genannt: 2-Amino-6-hydroxy-6-methyl-heptan, 2-Acetamido-6-hydroxy-6-methyl-heptan, 11-Amino-2,2,12-trimethyl-tridecan-1-ol, sowie 11-Aminoundecanole der in der deutschen Offenlegungsschrift 2831299 beschriebenen Art, soweit sie Reste der Formel II liefern.

Ausgangsprodukte der Formel V können auch durch Umsetzung eines Hydrochinons der Formel VII

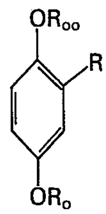

(VII)

mit einem zur Einführung von Gruppierungen der Formel III geeigneten Alkylierungsmittel erhalten werden. Dabei haben R die unter Formel I angegebene Bedeutung und $R_o$ und $R_{oo}$ die oben angegebene Bedeutung. Zur Alkylierung von Verbindungen der Formel VII eignen sich z.B. Isobutylen und Diisobutylen. Als Alkohole zur Alkylierung von Verbindungen der Formel VII kommen beispielsweise tert.-Butanol und 1,1,3,3-Tetramethylbutan-1-ol in Betracht.

Funktionelle Derivate von Verbindungen der Formel V oder VII können in Verbindungen mit anderen funktionellen Gruppen übergeführt werden. So können z.B. COOH-Gruppen Q mit Alkoholen $R_4OH$ zu den entsprechenden Estern verestert werden. Estergruppen Q $-COOR_4$ können einer Umesterung unterworfen oder durch Behandlung mit $NH(R_4)(R_5)$ in Amide $-CON(R_4)(R_5)$ übergeführt werden. Dabei haben $R_4$ und $R_5$ die

oben angegebene Bedeutung. Wenn Q eine Hydroxylgruppe ist, welche an eine Methylengruppe gebunden ist (beispielsweise wie $-CH_2OH$), kann diese Gruppe durch Behandlung mit Chromtrioxid zur Carboxylgruppe oxidiert werden.

Die Verbindungen der Formel I können zur Erzielung optimaler photographischer Effekte sehr unterschiedliche funktionelle Gruppen aufweisen. So können z.B. die Polarität oder der Ballast der 1,4-Benzochinone der Formel I je nach Bedarf eingestellt werden, um die gewünschte Löslichkeit, Verträglichkeit, Mobilität/Immobilität für photographische Systeme zu erzielen. Diese Eigenschaften machen die 1,4-Benzochinone der Formel I zu wertvollen Zwischenprodukten für die Herstellung von anderen Verbindungen für den Einsatz in photographischen Systemen. Zudem können die Verbindungen der Formel I als Bleichhemmer in Schichten photographischer Silberfarbbleichmaterialien eingesetzt werden.

In den folgenden Beispielen sind Teile und Prozente Gewichtsteile und Gewichtsprozente.

Beispiel 1

a) 110 Teile Hydrochinon, 284 Teile 5-Methylhex-5-en-säuremethylester und 10 Teile p-Toluolsulfonsäure werden während 24 Stunden auf einem Dampfbad erhitzt. Das abgekühlte Reaktionsgemisch verfestigt sich zum Teil und liefert nach Verreiben mit Diäthyläther und Filtration 2,5-Bis-(5'-methoxycarbonyl-2'-methylpent-2'-yl)-hydrochinon; Smp. 150–153°C. Nach dem Umkristallisieren aus Methanol/Wasser weist das Produkt einen Schmelzpunkt von 160–162°C und die folgende Elementaranalyse auf:

| | | |
|---|---|---|
| berechnet für $C_{22}H_{34}O_6$ | C 66,98 | H 8,69% |
| gefunden | C 67,05 | H 8,96% |

b) 25,0 Teile 2,5-Bis-(5'-methoxycarbonyl-2'-methylpent-2'-yl)-hydrochinon, 250 Teile Eisessig und 125 Teile 46%ige wässrige Bromwasserstoffsäure werden 15 Stunden bei Raumtemperatur gelagert. Danach wird das 2,5-Bis-(5'-carboxy-2'-methylpent-2'-yl)-hydrochinon, das 0,5 Molekül kristalliner Essigsäure enthält, abfiltriert; Smp. 221–224°C.

Elementaranalyse: berechnet für

| | | |
|---|---|---|
| $C_{20}H_{30}O_6 \cdot 1/2\ CH_3COOH$ | C 63,62 | H 8,14% |
| gefunden | C 63,68 | H 8,38% |

c) 44 Teile 2,5-Bis-(5'-carboxy-2'-methylpent-2'-yl)-hydrochinon werden unter kräftigem Rühren portionenweise zu 375 Teilen 10–14%iger wässriger Natriumhypochloritlösung gegeben, wobei die Temperatur mit einem kalten Wasserbad auf 25–30°C gehalten wird. Das Gemisch wird 1 Stunde bei Raumtemperatur gerührt, mit 300 Teilen Wasser verdünnt und mit konzentrierter Chlorwasserstoffsäure angesäuert. Nach dem Filtrieren erhält man 2,5-Bis-(5'-carboxy-2'-methylpent-2'-yl)-benzochinon, das nach dem Kristallisieren aus Methanol einen Smp. von 204–206°C aufweist.

Beispiel 2

10,8 Teile 2,5-Bis-(5'-carboxy-2'-methylpent-2'-yl)-1,4-benzochinon, 82 Teile n-Hexanol und 0,5 Teile p-Toluolsulfonsäure werden auf einem Dampfbad während 72 Stunden erhitzt. Überschüssiges n-Hexanol wird unter vermindertem Druck abgezogen. Der Rückstand wird in Diäthyläther aufgenommen und mit Natriumcarbonatlösung und dann mit Wasser gewaschen. Nach dem Verdampfen des Diäthyläthers erhält man aus der ätherischen Lösung durch Kurzwegdestillation bei 0,3 Millibar 2,5-Bis-(5'-n-hexyloxycarbonyl-2'-methylpent-2'-yl)-1,4-benzochinon als gelben Farbstoff; Smp. 45–47°C.

Elementaranalyse:

| | | |
|---|---|---|
| berechnet für $C_{32}H_{52}O_6$ | C 72,14 | H 9,84% |
| gefunden | C 72,15 | H 9,86% |

Beispiel 3

Analog zu Beispiel 2 wird aus Methanol und 2,5-Bis-(5'-carboxy-2'-methylpent-2'-yl)-1,4-benzochinon unter Verwendung von Chlorwasserstoff als Katalysator 2,5-Bis-(5'-methoxycarbonyl-2'-methylpent-2'-yl)-1,4-benzochinon hergestellt. Man erhält leuchtend gelbe Kristalle vom Smp. 85–87°C.

Elementaranalyse:

| | | |
|---|---|---|
| berechnet für $C_{22}H_{32}O_6$ | C 67,32 | H 8,22% |
| gefunden | C 67,56 | H 8,20% |

Beispiel 4

0,9 Teile 2,5-Bis-(5'-methoxycarbonyl-2'-methylpent-2'-yl)-1,4-benzochinon, 16,4 Teile n-Hexanol und 0,1 Teile p-Toluolsulfonsäure werden auf einem Dampfbad 6 Stunden erhitzt. Das Reaktionsgemisch wird mit Diäthyläther verdünnt, zuerst mit verdünnter Natriumhydroxidlösung und dann mit Wasser gewaschen und verdampft. Überschüssiges n-Hexanol wird unter vermindertem Druck (16 Millibar) abgezogen. Das zurückbleibende Produkt ergibt nach Kurzwegdestillation bei 0,2 Millibar 2,5-Bis-(5'-n-hexyloxycarbonyl-2'-methylpent-2'-yl)-1,4-benzochinon als gelben Farbstoff, der mit der Verbindung gemäss Beispiel 2 identisch ist.

Elementaranalyse:

| | | |
|---|---|---|
| berechnet für $C_{32}H_{52}O_6$ | C 72,14 | H 9,84% |
| gefunden | C 72,29 | H 9,73% |

Beispiel 5

10 Teile 2,5-Bis-(5'-methoxycarbonyl-2'-methylpent-2'-yl)-hydrochinon werden in 174 Teilen Chloroform gelöst. Zu dieser Lösung gibt man 1 Teil Tetrabutylammoniumhydrogensulfat in 125 Teilen 10–14%igem wässrigem Natriumhypochlorit. Das erhaltene Zweiphasensystem wird bei 25°C während 30 Minuten kräftig gerührt. Dann wird das Reaktionsgemisch mit Diäthyläther extrahiert und der Ätherextrakt mit Wasser gewaschen. Die ätherische Lösung wird unter vermindertem Druck abgezogen. Man erhält ein gelbes Öl. Dieses Öl wird in Methanol aufgenommen, filtriert und das Filtrat wird bei 25°C stehengelassen. Aus dieser methanolischen Lösung kristallisiert das 2,5-Bis-(5'-methoxycarbonyl-2'-methylpent-2'-yl)-1,4-benzochinon in Form gelber Prismen aus. Es ist identisch mit der Verbindung gemäss Beispiel 3.

Beispiel 6

a) 12,4 Teile 2-Methylhydrochinon, 14,2 Teile 5-Methyl-hex-5-en-methylester und 0,5 Teile p-Toluolsulfonsäure werden auf einem Dampfbad 24 Stunden erhitzt. Das abgekühlte Reaktionsgemisch wird mit Diäthyläther behandelt, dann zuerst mit einer 10%igen wässrigen Natriumhydroxidlösung und dann mit Wasser gewaschen, bis die wässrige Phase neutral ist. Die ätherische Phase wird abgetrennt und verdampft. Man erhält ein viskoses Öl, das nach Kurzwegdestillation bei 0,1 Millibar 5-(2',5'-Dihydroxy-4'-methylphenyl)-5-methylhexensäuremethylester liefert.

Elementaranalyse:

| | | |
|---|---|---|
| berechnet für $C_{15}H_{22}O_4$ | C 67,64 | H 8,32% |
| gefunden | C 67,37 | H 8,27% |

b) Analog dem in Beispiel 5 beschriebenen Verfahren wird unter Verwendung von 5-(2',5'-Dihydroxy-4'-methylphenyl)-5-methylhexensäuremethylester das 2-(5'-n-Hexyloxycarbonyl-2'-methylpent-2'-yl)-5-methyl-1,4-benzochinon als Öl erhalten, dessen Zusammensetzung mit dem für $C_{20}H_{30}O_4$ berechneten Wert übereinstimmt.

Beispiel 7

Analog dem in Beispiel 5 beschriebenen Verfahren wird aus 5-(4'-tert-Butyl-2',5'-dihydroxyphenyl)-5-methyl-hexansäure das 2-tert-Butyl-5-(5'-carboxy-2'-methylpent-2'-yl)-1,4-benzochinon in Form gelber Prismen hergestellt; Smp. 143–146°C.

Beispiel 8

5,5 Teile Hydrochinon, 21,2 Teile 5-Methyl-hex-5-en-säuremethylester und 1,0 Teil p-Toluolsulfonsäure werden auf einem Dampfbad 4 Tage erhitzt. Das abgekühlte Reaktionsgemisch wird in Diäthyläther aufgenommen, in 10%iger wässriger Natriumhydroxidlösung und dann mit Wasser gewaschen, bis die wässrige Phase neutral ist. Nach dem Abziehen des Diäthyläthers wird das teilweise verfestigte zurückbleibende Öl mit Petroläther (Siedebereich 40–60°C) verrieben. Man erhält 2,5-Bis-(5'-n-hexyloxycarbonyl-2'-methylpent-2'-yl)-hydrochinon; Smp. 77–78°C. Nach weiterer Kristallisation aus Petroläther mit einem Siedebereich von 60–80°C weist das Produkt einen Smp. von 83–86°C auf. Unter Verwendung von 2,5-Bis-(5'-n-hexyloxycarbonyl-2'-methylpent-2'-yl)-hydrochinon wird analog Beispiel 5 das 2,5-Bis-(5'-n-hexyloxycarbonyl-2'-methylpent-2'-yl)-1,4-benzochinon hergestellt, das mit dem Produkt gemäss Beispiel 2 identisch ist.

Beispiel 9

2,0 Teile 2,5-Bis-(5'-n-heptyloxycarbonyl-2'-methyl-pent-2'-yl)-hydrochinon in 25 Teilen Aceton werden mit dem Jones Reagenz (zu dessen Herstellung siehe Bowers, Halsall, Jones und Lemin, J.Chem. Soc., 2555, 1953) bis zur vollständigen Oxidation, welche durch Anzeige einer dauernden braunen Farbe charakterisiert ist, verrieben. Nach Verdünnen mit Wasser wird das Reaktionsgemisch mit Diäthyläther extrahiert, die ätherische Phase mit Wasser gewaschen und dann eingedampft. Nach Kurzwegdestillation bei 0,6 Millibar wird 2,5-Bis-(5'-n-hexyloxycarbonyl-2'-methylpent-2'-yl)-1,4-benzochinon als gelbes Öl erhalten.

In folgender Tabelle 1 werden weitere Benzochinone aufgeführt, welche analog dem in Beispiel 9 beschriebenen Verfahren hergestellt werden.

Tabelle 1

| Beispiel Nr. | In 2,5-Stellung substituierte 1,4-Benzochinone | Siedep. in °C/mBar oder Schmelzp. (°C) |
|---|---|---|
| 10 | Bis-5-(2'-äthylhexyloxycarbonyl-2-methyl-pent-2-yl) | 250/0.6 |
| 11 | Bis-(5-cyclohexyloxycarbonyl-2-methyl-pent-2-yl) | 136–138 |
| 12 | cis/trans-Bis-(4-methoxycarbonyl-1-methyl-cyclohex-1-yl) | 160–170 |
| 13 | Bis-(8-acetyloxy-2,6-dimethyl-oct-2-yl) | 250/1 |
| 14 | Bis-(8-n-butoxy-2,6-dimethyl-oct-2-yl) | 250/0.6 |
| 15 | 2-t-Butyl-dimethyl-5-(2-methyl-but-2-yl-4-phosphonate) | 105–107 |
| 16 | 2-t-Butyl-dimethyl-5-(5-methoxycarbonyl-2-methyl-hex-2-yl-5-phosphonate) | 69–71 |
| 17 | Bis-(5-isopropyloxycarbonyl-2-methyl-pent-2-yl) | Öl |

Beispiel 18

2,0 Teile 2,5-Bis-(2',6'-dimethyl-8'-hydroxy-oct-2'-yl)-hydrochinon in 20 Teilen Aceton werden mit dem Jones Reagenz oxidiert und gemäss dem in Beispiel 9 angegebenen Verfahren ausgearbeitet. Man erhält eine Säure 2,5-Bis-(2',6'-dimethyl-7'-carboxy-hept-2'-yl)-1,4-benzochinon, welche nach Veresterung mit Methanol und Kurzwegdestillation bei 0,7 Millibar den entsprechenden Ester liefert.

Beispiel 19

a) 100 Teile 98%ige Schwefelsäure werden zu 32 Teilen Methanol bei einer Temperatur unter 10°C zugegeben. Dazu werden 35,5 Teile 5-Methylhex-5-ensäuremethylester und danach 12,4 Teile Hydrochinon-monomethyläther gegeben. Nach Rühren während 24 Stunden bei Raumtemperatur wird die Reaktionsmischung in Wasser gegossen und die erhaltene ölige Schicht mit Diäthyläther extrahiert. Nach Waschen mit einer 2N Natriumhydroxid-Lösung und dann mit Wasser wird die ätherische Phase eingedampft. Der erhaltene ölige Rückstand wird mit Petroläther (40–60°C), enthaltend ein wenig Diäthyläther, verdünnt. Man erhält 2,5-Bis-(5'-methoxy-carbonyl-2'-methylpent-2'-yl)-4-methoxyphenol vom Schmelzpunkt 82–84°C und mit folgender Elementaranalyse:

| | | |
|---|---|---|
| berechnet für $C_{23}H_{36}O_6$ | C 67,62 | H 8,88% |
| gefunden | C 67,99 | H 9,16% |

b) 4,1 Teile 2,5-Bis-(5'-methoxycarbonyl-2'-methyl-pent-2'-yl)-4-methoxyphenol in 30 Teilen Aceton werden mit dem Jones Reagenz oxidiert und gemäss dem in Beispiel 9 beschriebenen Verfahren ausgearbeitet. Nach Kristallisation aus Methanol und Wasser erhält man 2,5-Bis-(5'-methoxycarbonyl-2'-methyl-pent-2'-yl)-1,4-benzochinon vom Smp. 84–86°C, das mit dem in Beispiel 3 erhaltenen Produkt identisch ist.

Beispiel 20

a) Zu 13,6 Teilen $AlCl_3$ ist 25 Teilen Nitromethan werden 13,8 Teile Hydrochinondimethyläther und 14,2 Teile 5-Methyl-hex-5-ensäuremethylester (gelöst in 25 Teilen Nitromethan) bei Raumtemperatur zugetropft. Nach Stehenlassen bei Raumtemperatur während 2 Tagen wird das Reaktionsgemisch in Wasser gegossen und die organische Phase mit Äthyläther extrahiert. Der ätherische Auszug wird zuerst mit Wasser, dann mit einer 2N Natriumhydroxid-Lösung und anschliessend wiederum mit Wasser gewaschen und danach eingedampft. Man erhält 2,5-Bis-(5'-methoxycarbonyl-2'-methyl-pent-2'-yl)-1,4-dimethoxybenzol bei 220°C/0,4 Millibar vom Smp. 81–83°C nach Kristallisation aus Petroläther (40–60°C).

Elementaranalyse:

| | | |
|---|---|---|
| berechnet für $C_{24}H_{38}O_6$ | C 68,22 | H 9,06% |
| gefunden | C 68,48 | H 9,12% |

b) Analog zum in Beispiel 9 angegebenen Oxidationsverfahren wird 2,5-Bis-(5'-methoxycarbonyl-2'-methyl-pent-2'-yl)-1,4-dimethoxybenzol zu 2,5-Bis-(5'-methoxycarbonyl-2'-methyl-pent-2'-yl)-1,4-benzochinon oxidiert, das mit dem gemäss Beispiel 3 erhaltenen Produkt identisch ist.

Beispiel 21

a) 62,0 Teile Hydrochinon-monomethyläther, 28,4 Teile 5-Methyl-hex-5-ensäuremethylester und 2,0 Teile p-Toluolsulfonsäure werden auf einem Wasserdampfbad während 5 Tagen geheizt. Das Reaktionsgemisch wird danach mit Diäthyläther verdünnt und zuerst mit einer 2N Natriumhydroxidlösung und dann mit Wasser gewaschen. Nach Eindampfen des Äthers wird der Rückstand destilliert. Man erhält 2-(5'-Methoxycarbonyl-2'-methyl-pent-2'-yl)-4-methoxyphenol mit Siedepunkt 154°C/0,1 Millibar.

Elementaranalyse:

| | | |
|---|---|---|
| berechnet für $C_{15}H_{22}O_4$ | C 67,65 | H 8,33% |
| gefunden | C 67,92 | H 8,56% |

b) 9,0 Teile 2-(5-Methoxycarbonyl-2'-methyl-pent-2'-yl)-4-methoxyphenol in 50 Teilen Aceton werden mit dem Jones Reagenz oxydiert. Man erhält 2-(5'-Methoxycarbonyl-2'-methyl-pent-2'-yl)-1,4-benzochinon mit Siedepunkt 140–144°C/0,9 Millibar.

Elementaranalyse:

| | | |
|---|---|---|
| berechnet für $C_{14}H_{18}O_4$ | C 67,18 | H 7,25% |
| gefunden | C 67,10 | H 7,30% |

Beispiel 22

Auf einen transparenten Cellulosetriacetatträger wird eine Schicht aufgetragen, die pro $m^2$: 12g Gelatine, 0,26g Farbstoff der Formel

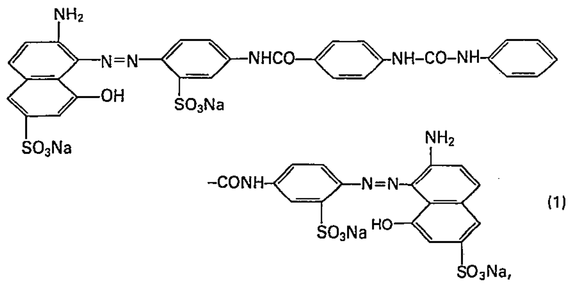

(1)

0,59g Silber (als Silberbromiddispersion), 0,80g 1,3-Dichlortriazin-5-aminobenzol-4'-sulfonsäure als Härter für die Gelatine und 0,17g einer feinteiligen Dispersion der Verbindung der Formel

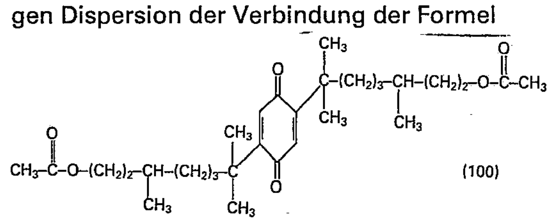

(100)

als Bleichhemmer enthält.

Das gleiche photographische Element wird hergestellt, jedoch ohne Zusatz des Bleichhemmers.

Je zwei Streifen dieser Elemente werden durch einen Stufenkeil während einer Sekunde mit 500 Lux belichtet. Anschliessend wird je 1 Streifen der Elemente bei 24°C folgendermassen behandelt:

| | | | |
|---|---|---|---|
| (A) | 1. Entwickeln | 8 Minuten | Bad 1 |
| | 2. Bleichen | 4 Minuten | Bad 2 |
| | 3. Fixieren | 3 Minuten | Bad 3 |
| | 4. Wässern | 3 Minuten | |

Die beiden übrigen Streifen werden wie folgt verarbeitet:

| | | | |
|---|---|---|---|
| (B) | 1. Entwickeln | 8 Minuten | Bad 1 |
| | 2. Fixieren | 3 Minuten | Bad 3 |
| | 3. Wässern | 3 Minuten | |

Die Bäder haben die folgende Zusammensetzung:

Bad 1: Entwickler

| | |
|---|---|
| Ethylendiamin-tetraessigsäure (Natriumsalz) | 2g |
| Kaliumhydroxid 85% | 22g |
| Kaliummetabisulfit | 15g |
| Borsäure | 13,35g |
| Kaliumbromid | 1,65g |
| Benztriazol | 0,25g |
| Phenidon | 2,5g |
| Hydrochinon | 8,35g |
| Ascorbinsäure | 8,35g |
| Wasser auf | 1000 ml |

Bad 2: Bleichbad

| | |
|---|---|
| m-Nitrobenzolsulfonsäure | 12g |
| Sulfaminsäure | 280g |
| Ethylcellosolve | 120ml |
| 2,3-Dimethylchinoxalin | 4g |
| Kaliumiodid | 12g |
| Wasser auf | 1000ml |

Bad 3: Fixierbad

| | |
|---|---|
| Ethylendiamin-tetraessigsäure (Natriumsalz) | 3 g |
| Ammoniumthiosulfat 60% | 333 g |
| Ammoniumbisulfit 60% | 75 g |
| Ammoniak | 21 ml |
| Wasser auf | 1000 ml |

Man erhält nach (A) für beide Elemente einen klaren, scharfen Magentakeil und nach (B) jeweils einen Silberkeil, der vom eingegossenen Farbstoff überlagert ist.

Die Magentadichte der nach (A) erhaltenen Keile wird mittels eines Remissionsdensitometers im grünen Spektralbereich gemessen. Die maximale Magentadichte beträgt 1,80.

Die Silberdichte wird mit dem gleichen Instrument im roten Spektralbereich bestimmt:

Das von Bleichhemmer freie Element, das eine Silbermenge der Dichte 0,5 enthält, kann den Magentafarbstoff bis zu einer Dichte von 0,5 ausbleichen. Enthält das Element dagegen einen Bleichhemmer, wird mit derselben Silbermenge der Magentafarbstoff nur bis zur Dichte 1,25 ausgebleicht [die Farbbleichung wird durch die Verbindung der Formel (100) gehemmt].

Verwendet man anstelle der Verbindung der Formel (100) als Bleichhemmer gleiche Mengen der Verbindungen der Formeln

(101)

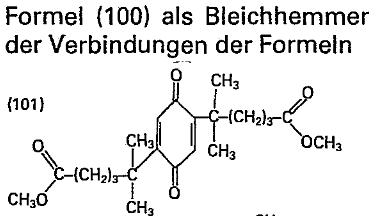

(102)

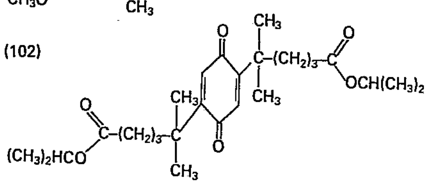

(103)

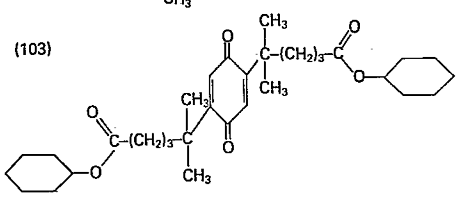

,

(104)

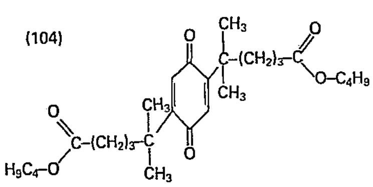

oder

(105)

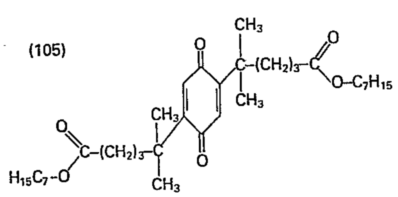

,

so erhält man nach Belichtung und Verarbeitung folgende Magentadichten:

| | |
|---|---|
| Verbindung (101) | 1,20 |
| Verbindung (102) | 1,05 |
| Verbindung (103) | 1,30 |
| Verbindung (104) | 1,00 |
| Verbindung (105) | 0,90 |

Beispiel 23

Es werden vier photographische Elemente hergestellt, die ein negatives Cyanbild nach dem Silberfarbbleichverfahren ergeben:

Element A: Auf einen transparenten Polyesterträger werden folgende Schichten aufgetragen:

eine Schicht, die pro $m^2$ 1,6 g Gelatine, 0,135 g blaugrünen Farbstoff der Formel

(2)

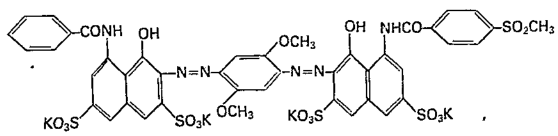

,

0,05 mg rote, kolloidale Goldteilchen als Entwicklungskeime und 40 mg 1-Amino-3-hydroxy-5-methylmorpholiniumtriazintetrafluoroborat als Härter enthält;

eine Schicht, die pro $m^2$ 1 g Gelatine, 0,5 g Verbindung der Formel (100) und 20 mg besagten Härters enthält, und

eine Schicht, die pro $m^2$ 1,8 g Gelatine, 0,4 g Silber als rotempfindliche Silberchlorobromidemulsion mit 25 Mol% Silberchlorid und einer mittleren Teilchengrösse von 0,3 µm, und 40 mg besagten Härters enthält.

Element B wird wie Element A hergestellt, jedoch ohne Bleichhemmer in der zweiten Schicht.

Element C wird wie Element A hergestellt, jedoch ohne die zweite Schicht.

Element D wird wie Element C hergestellt, in der dritten Schicht sind jedoch 1 g/$m^2$ der Verbindung der Formel (100) enthalten.

Proben der Elemente A bis D werden in einem Sensitometer mit rotem Licht belichtet und anschliessend wie folgt verarbeitet:

1. Silberentwicklung während 3 Minuten bei 30°C in einem Bad der folgenden Zusammensetzung:

| | |
|---|---|
| Äthylendiamintetraessigsäure (Natriumsalz) | 4 g |
| Kaliumsulfit | 19,9 g |
| Natriumsulfit, wasserfrei | 38,0 g |
| Hydrochinon | 8,0 g |
| 1-Phenyl-3-methyl-5-pyrazolidon | 0,5 g |
| Kaliumkarbonat, wasserfrei | 19,5 g |
| Kaliumbikarbonat | 13,3 g |
| Kaliumbromid | 3,5 g |
| Benztriazol | 1,0 g |
| Äthylcellosolve | 57,4 g |
| Natriumthiosulfat, wasserfrei | 0,9 g |
| Wasser bis | 1000 ml |

Es entsteht ein negatives Silberbild in der dritten Schicht und durch Silberkomplexdiffusion und

Silberabscheidung an den Entwicklungskeimen ein positives Silberbild in der ersten Schicht.

2. Wässern während 1 Minute.

3. Gleichzeitige Farbstoff- und Silberbleichung während 3 Minuten bei 30°C in einem Bleichbad der folgenden Zusammensetzung:

| | |
|---|---|
| Schwefelsäure (100%) | 41,8 g |
| m-Nitrobenzosulfonsäure, (Natriumsalz) | 7,5 g |
| Äthylcellosolve | 57,4 g |
| 2,3,6-Trimethylchinoxalin | 1,1 g |
| Kaliumjodid | 9,0 g |
| Bis-(β-Cyanoäthyl)-sulfoäthylphosphin, (Natriumsalz) | 2,9 g |
| Wasser bis | 1000 ml |

4. Wässern während 1 Minute.

5. Fixieren während 3 Minuten bei 30°C in einem Fixierbad der in Beispiel 1 angegebenen Zusammensetzung.

6. Wässern während 4 Minuten und trocknen der Elemente.

Alle vier Elemente ergeben ein negatives, d.h. zum Belichtungskeil gegenläufiges Farbbild. Misst man darin die maximale Farbstoffdichte, so erhält man die folgenden Werte

| Element | A | B | C | D |
|---|---|---|---|---|
| Maximaldichte | 1,0 | 0,58 | 0,35 | 0,80 |

Vergleicht man die Maximaldichten der vier Elemente mit der Maximaldichte von entsprechenden Proben, die direkt nach der Entwicklung fixiert werden (100%), so ergibt sich für A bis D demgegenüber folgender Farbstoffverlust:

| Element | A | B | C | D |
|---|---|---|---|---|
| Farbstoffverlust | 0% | 42% | 45% | 20% |

**Patentansprüche**

1. 1,4-Benzochinone der Formel I

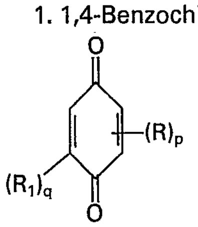 (I),

worin p 1 oder 2 und q 0 oder 1 sind, mit der Massgabe, dass p+q 1 oder 2 ist,

R einen Rest der Formel II

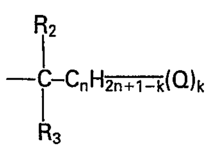 (II),

und

Q $-COOR_4$, $-CON(R)_4)(R_5)$, $-OR_5$, $-OCOR_7$, $-N(R_8)(R_9)$, $-PO(OR_{10})([O]_xR_{11})$, $-SO_2R_{12}$, $-CN$, Halogen, $-NO_2$ oder $-COR_{13}$ bedeuten,

n eine ganze Zahl von 1 bis 20,

k die Zahl 1 oder 2 und

x die Zahl 0 oder 1 sind,

$R_1$ $C_{1-8}$-Alkyl oder ein Rest der Formel II ist, wobei durch R und $R_1$ dargestellte Reste der Formel II gleich oder verschieden sein können, und

$R_2$ und $R_3$ unabhängig voneinander $C_{1-5}$-Alkyl sind, wobei, wenn Q $-COOR_4$ darstellt, eines von $R_2$ und $R_3$ durch eine Gruppe $-COOR_4$ substituiert sein kann, oder $R_2$ oder $R_3$ so mit dem Rest $-C_nH_{2n+1-k}$ verbunden sein können, dass ein durch die Gruppe (n) $-(COOR_4)_k$ mit gleichen oder verschiedenen $R_4$ substituierter $C_{5-12}$-Cycloalkylenrest gebildet wird,

$R_4$ Wasserstoff, $C_{1-20}$-Alkyl, das durch 1–5 Sauerstoffatome unterbrochen und durch eine Gruppe $-OR_6$ substituiert sein kann, $C_{3-20}$-Alkenyl, $C_{3-12}$-Cycloalkyl, $C_{6-20}$-Aryl, das durch eine $C_{1-4}$-Alkylgruppe substituiert sein kann, $C_{7-13}$-Aralkyl, einen 5- oder 6-gliedrigen, Sauerstoffhaltigen Heterocyclus, der durch eine oder zwei $C_{1-4}$-Alkylgruppen substituiert sein kann, oder Methyl darstellt, das durch einen 5- oder 6-gliedrigen, ein Sauerstoffatom enthaltenden, unsubstituierten oder durch eine oder zwei $C_{1-4}$-Alkylgruppen substituierten Heterocyclus substituiert ist,

$R_5$ Wasserstoff oder $C_{1-20}$-Alkyl bedeutet oder $R_4$ und $R_5$ zusammen mit dem Stickstoffatom, an das sie gebunden sind, einen 5- oder 6-gliedrigen Heterocyclus bilden, der durch eine oder zwei $C_{1-4}$-Alkylgruppen substituiert sein kann,

$R_6$ $C_{3-12}$-Cycloalkyl, $C_{3-20}$-Alkenyl, $C_{6-10}$-Aryl, das durch eine oder zwei $C_{1-4}$-Alkylgruppen substituiert sein kann, oder $C_{7-13}$-Aralkyl bedeutet, und

$R_7$ Wasserstoff, $C_{1-20}$-Alkyl, $C_{3-20}$-Alkenyl, $C_{3-12}$-Cycloalkyl, $C_{7-13}$-Aralkyl oder $C_{6-10}$-Aryl darstellt, das durch eine oder zwei $C_{1-4}$-Alkylgruppen substituiert sein kann,

$R_8$ Wasserstoff oder $C_{1-4}$-Alkyl und

$R_9$ Wasserstoff, $C_{1-4}$-Alkyl oder eine Gruppe $-COR_7$ bedeuten, wobei $R_7$ die oben angegebene Bedeutung hat, oder $R_8$ und $R_9$ zusammen mit dem Stickstoffatom, an das sie gebunden sind, einen substituierten oder durch eine oder zwei $C_{1-4}$-Alkylgruppen substituierten 5- oder 6-gliedrigen Ring bilden, und

$R_{10}$ und $R_{11}$ bei x=1 unabhängig voneinander Wasserstoff, $C_{1-20}$-Alkyl oder zusammen $C_{2-3}$-Alkylen darstellen, das durch 1–4 $C_{1-20}$-Alkylgruppen substituiert sein kann, und bei x=0 $R_{10}$ Wasserstoff oder $C_{1-20}$-Alkyl und $R_{11}$ geradkettiges $C_{1-5}$-Alkyl bedeuten,

$R_{12}$ $-OH$, $-Cl$ oder $-N(R_5)(R_7)$ bedeutet, und

$R_{13}$ $-H$, $C_{1-20}$-Alkyl oder Halogen bedeutet, wobei $R_5$ und $R_7$ die oben angegebene Bedeutung haben, mit der Massgabe, dass $R_1$ einen Rest der Formel II darstellt, wenn $R_{12}$–OH ist,

sowie Salze von Verbindungen der Formel I mit organischen oder anorganischen Säuren und Basen.

2. Benzochinone der Formel I nach Anspruch 1, worin die Gruppen R und $R_1$ in 2- und 5-Stellung des 1,4-Benzochinons stehen.

3. Benzochinone der Formel I nach Anspruch I, in welcher $R_1$ einen Rest der Formel III

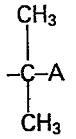

(III)

bedeutet, worin A Alkyl mit 1 bis 5 Kohlenstoffatomen ist.

4. Benzochinone der Formel I nach Anspruch 1, in welcher R ein Rest der Formel II ist, worin Q $-COOR_4$, $-OCOR_7$ oder $-CN$ ist, wobei $R_1$ bis $R_4$, $R_7$, p, q, k und n die in Anspruch 1 angegebenen Bedeutungen haben.

5. Benzochinone nach Anspruch 4, dadurch gekennzeichnet, dass in der Verbindung der Formel I R und $R_1$ ein Rest der Formel II sind, worin Q $-COOR_4$ ist, wobei $R_4$ $C_{1-12}$-Alkyl, das unsubstituiert oder durch $C_{3-12}$-Cycloalkyloxy oder $C_{6-10}$-Aryloxy substituiert ist, $C_{3-12}$-Cycloalkyl, $C_{6-10}$-Aryl, $C_{7-13}$-Aralkyl oder Methyl darstellt, das durch einen 5- oder 6-gliedrigen, ein Sauerstoffatom enthaltenden, unsubstituierten, heterocyclischen Ring substituiert ist, oder Q $-OCOR_7$ ist, worin $R_7$ Wasserstoff, $C_{1-20}$-Alkyl, $C_{3-12}$-Cycloalkyl, $C_{7-13}$-Aralkyl oder unsubstituiertes $C_{6-10}$-Aryl bedeutet, $R_2$ und $R_3$ unabhängig voneinander Alkyl mit 1 bis 5 Kohlenstoffatomen sind, p und q 1 sind und k und n die in Anspruch 4 angegebene Bedeutung haben.

6. Benzochinone nach Anspruch 5, dadurch gekennzeichnet, dass sie der Formel IV

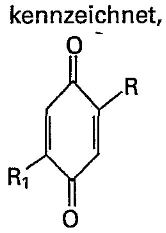

(IV),

entsprechen, in welcher R und $R_1$ ein Rest der Formel II sind, worin Q $-COOR_4$, wobei $R_4$ Alkyl mit 1 bis 8 Kohlenstoffatomen oder Cycloalkyl mit 5 bis 8 Kohlenstoffatomen ist, oder $-OCOR_7$ ist, worin $R_7$ Wasserstoff oder Alkyl mit 1 bis 4 Kohlenstoffatomen ist, $R_2$ und $R_3$ jeweils Methyl, k 1 und n 1 bis 10 sind.

7. Benzochinone der Formel IV nach Anspruch 6, in welcher n die Zahl 3 ist, $R_2$ und $R_3$ Methyl und Q $-COOR_4$ bedeuten, wobei $R_4$ Alkyl mit 1 bis 8 Kohlenstoffatomen ist.

8. Benzochinone der Formel I nach Anspruch 1, in welcher Q ein Rest $-COOR_4$, $-CON(R_4)(R_5)$, $-OR_5$, $-OCOR_7$, $-N(R_8)(R_9)$, $-OPO(OR_{10})([0]_xR_{11})$, $-SO_2R_{12}$ oder $-CN$ ist, k, p, q, n, R, $R_1$ bis $R_3$, $R_5$ bis $R_{12}$ und x die im Anspruch 1 angegebene Bedeutung haben und $R_4$ $-H$, $C_{1-20}$-Alkyl, das durch 1–5 Sauerstoffatome unterbrochen und durch eine Gruppe $-OR_6$ substituiert sein kann, wobei $R_6$ die oben angegebene Bedeutung hat, ferner $R_4$ $C_{3-20}$-Alkenyl, $C_{3-12}$-Cycloalkyl, $C_{6-10}$-Aryl, das durch eine $C_{1-4}$-Alkylgruppe substituiert sein kann, $C_{7-13}$-Aralkyl, einen 5- oder 6-gliedrigen, sauerstoffhaltigen Heterocyclus, der durch eine oder zwei $C_{1-4}$-Alkylgruppen substituiert sein kann, oder Methyl darstellt, das durch einen 5- oder 6-gliedrigen, ein Sauerstoffatom enthaltenden, substituierten oder durch eine oder zwei $C_{1-4}$-Alkylgruppen substituierten Heterocyclus substituiert ist.

**Claims**

1. Benzo-1,4-quinones of the formula

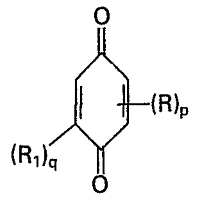

(I),

wherein p is 1 or 2 and q is 0 or 1, with the proviso that p+q is 1 or 2;

R is a radical of formula II

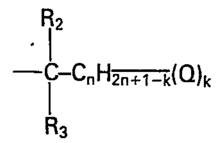

(II),

and

Q is selected from the radicals $-COOR_4$, $-CON(R_4)(R_5)$, $-OR_5$, $-OCOR_7$, $-N(R_8)(R_9)$, $-OPO(OR_{10})([O]_xR_{11})$, $-SO_2R_{12}$, $-CN$, halogen, $-NO_2$ or $-COR_{13}$,

n is an integer from 1 to 20,

k is 1 or 2 and

x is 0 or 1,

$R_1$ is $C_1$–$C_8$ alkyl or a radical of formula II, which radicals of formula II represented by $R_1$ and R may be the same or different;

$R_2$ and $R_3$ are each independently of the other $C_1$–$C_5$ alkyl and, when Q is $-COOR_4$, either $R_2$ and $R_3$ may be substituted by a $-COOR_4$ group, or $R_2$ or $R_3$ may be linked to the radical $C_nH_{2n+l-k}$ so as to form a $C_5$–$C_{12}$ cycloalkylene radical which is substituted by the group or groups $-(COOR_4)_k$, in which the $R_4$ groups are the same or different, and

$R_4$ is hydrogen, $C_1$–$C_{20}$ alkyl which may be interrupted by 1 to 5 oxygen atoms and substituted by a group $-OR_6$, or is $C_3$–$C_{20}$ alkenyl, $C_3$–$C_{12}$ cycloalkyl, $C_6$–$C_{10}$ aryl, which may be substituted by a $C_1$–$C_4$ alkyl group; or is $C_7$–$C_{13}$ aralkyl, a 5- or 6-membered heterocycle containing an oxygen atom and which may be substituted by one or two $C_1$–$C_4$ alkyl groups; or methyl substituted by a 5- or 6-membered heterocycle containing an oxygen atom and which may be substituted by one or two $C_1$–$C_4$ alkyl groups;

$R_5$ is hydrogen or $C_1$–$C_{20}$ alkyl, or $R_4$ and $R_5$ together with the nitrogen atom to which they are each attached form a 5- or 6-membered heterocyclic ring which may be substituted by one or two $C_1$–$C_4$ alkyl groups;

$R_6$ is $C_3$–$C_{12}$ cycloalkyl, $C_3$–$C_{20}$ alkenyl, $C_6$–$C_{10}$ aryl which may be substituted by one or two $C_1$–$C_4$ alkyl groups, or is $C_7$–$C_{13}$ aralkyl, and

$R_7$ is hydrogen, $C_1$–$C_{20}$ alkyl, $C_3$–$C_{20}$ alkenyl, $C_3$–$C_{12}$ cycloalkyl, $C_7$–$C_{13}$ aralkyl or $C_6$–$C_{20}$ aryl

which may be substituted by 1 or 2 $C_{1-4}$ alkyl groups;

$R_8$ is hydrogen or $C_1$–$C_4$ alkyl;

$R_9$ is hydrogen, $C_1$–$C_4$ alkyl or an acyl group of formula $-COR_7$, wherein $R_7$ is as defined above; or $R_8$ and $R_9$, together with the nitrogen atom to which they are each attached, form a 5- or 6-membered ring which is unsubstituted or substituted by one or two $C_1$–$C_4$ alkyl groups, and when x is 1,

$R_{10}$ and $R_{11}$ are each independently hydrogen, $C_1$–$C_{20}$ alkyl, or $R_{10}$ and $R_{11}$ together form a $C_2$–$C_3$ alkylene chain which may be substituted by one to four $C_1$–$C_{20}$ alkyl groups; and when x is 0, $R_{10}$ is hydrogen or $C_1$–$C_{20}$ alkyl, and $R_{11}$ is a $C_1$–$C_5$ straight chain alkyl group, and

$R_{12}$ is $-OH$, $-Cl$ or $-N(R_5)(R_7)$ wherein $R_5$ and $R_7$ are as defined above,

$R_{13}$ is hydrogen, $C_1$–$C_{20}$ alkyl or halogen, with the proviso that, when $R_{12}$ is $-OH$, then $R_1$ is a radical of formula II,

and salts thereof with organic or inorganic acids and bases.

2. Benzoquinones of formula I according to claim 1 wherein the groups R and $R_1$ are bonded in the 2- and 5-positions, respectively, in the benzo-1,4-quinones of formula I.

3. Benzoquinones of formula I according to claim 1, wherein $R_1$ is a group of formula III

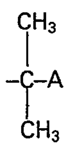

(III)

wherein A is an alkyl group having from 1 to 5 carbon atoms.

4. Benzoquinones of formula I according to claim 1, wherein R is a group of formula II, wherein Q is $-COOR_4$, $-OCOR_7$ or $-CN$ and $R_1$, $R_2$, $R_3$, $R_4$ and $R_7$, p, q, k and n are as defined in claim 1.

5. Benzoquinones according to claim 4, wherein R and $R_1$ are a group of formula II, wherein Q is $-COOR_4$ and $R_4$ is $C_1$–$C_{12}$ alkyl which is unsubstituted or substituted by $C_3$–$C_{12}$ cycloalkyloxy or by $C_6$–$C_{10}$ aryloxy, or $R_4$ is $C_3$–$C_{12}$ cycloalkyl, $C_6$–$C_{10}$ aryl, $C_7$–$C_{13}$ aralkyl or a methyl group substituted by a 5- or 6-membered unsubstituted heterocyclic ring containing an oxygen atom, or Q is $-OCOR_7$, wherein $R_7$ is hydrogen, $C_1$–$C_{20}$ alkyl, $C_3$–$C_{12}$, cycloalkyl, $C_7$–$C_{13}$ aralkyl or unsubstituted $C_6$–$C_{10}$ aryl, $R_2$ and $R_3$ are each independently hydrogen or $C_1$–$C_5$ alkyl, p and q are 1 and k and n are as defined in claim 4.

6. Benzoquinones according to claim 5 of the formula IV

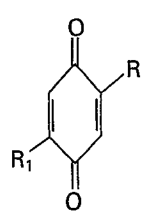

(IV),

wherein R and $R_1$ are a group of formula II, wherein Q is $-COOR_4$ and $R_4$ is $C_1$–$C_8$ alkyl or $C_5$–$C_8$ cycloalkyl, or Q is $-OCOR_7$, wherein $R_7$ is hydrogen or $C_1$–$C_4$ alkyl, $R_2$ and $R_3$ are each methyl, k is 1 and n is an integer from 1 to 10.

7. Benzoquinones of the formula IV according to claim 6, wherein n is 3, $R_2$ and $R_3$ are each methyl, Q is $-COOR_4$ and $R_4$ is $C_1$–$C_8$ alkyl.

8. Benzoquinones of formula I according to claim 1, wherein Q is selected from the radicals $-COOR_4$, $-CON(R_4)(R_5)$, $-OR_5$, $-OCOR_7$, $-N(R_8)(R_9)$, $-OPO(OR_{10})([0]_xR_{11})$, $-SO_2R_{12}$ or $-CN$, k, p, q, n, R, $R_1$ to $R_3$, $R_5$ to $R_{12}$ and x are as defined in claim 1 and $R_4$ is hydrogen, $C_1$–$C_{20}$ alkyl which may be interrupted by 1 to 5 oxygen atoms and substituted by a group $-OR_6$ wherein $R_6$ is as defined in claim 1, or $R_4$ is $C_3$–$C_{20}$ alkenyl, $C_3$–$C_{12}$ cycloalkyl, $C_6$–$C_{10}$ aryl which may be substituted by a $C_1$–$C_4$ alkyl group; or is $C_7$–$C_{13}$ aralkyl, a 5- or 6-membered heterocycle containing an oxygen atom and which may be substituted by one or two $C_1$–$C_4$ alkyl groups; or is methyl which is substituted by a 5- or 6-membered heterocyclic containing an oxygen atom and which is unsubstituted or substituted by one or two $C_1$–$C_4$ alkyl groups.

**Revendications**

1. 1,4-benzoquinones de formule I

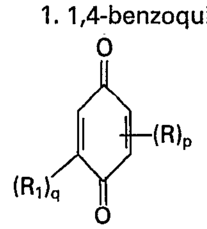

(I),

dans laquelle p vaut 1 ou 2 et q vaut 0 ou 1, à la condition que p+q soit égal à 1 ou 2,

R est un radical de formule II

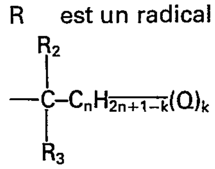

(II),

Q est un radical $-COOR_4$, $-CON(R_4)(R_5)$, $-OR_5$, $-OCOR_7$, $-N(R_8)(R_9)$, $-PO(OR_{10})([0]_xR_{11})$, $-SO_2R_{12}$, $-CN$, halogène, $-NO_2$ ou $-COR_{13}$,

n est un nombre entier de 1 à 20,

k est le nombre 1 ou 2, et

x est le nombre 0 ou 1,

$R_1$ est un radical alkyle en $C_{1-8}$ ou un radical de formule II, les radicaux de formule II représentés par R et $R_1$ pouvant être identiques ou différents, et

$R_2$ et $R_3$, indépendamment l'un de l'autre, sont chacun un radical alkyle en $C_{1-5}$ et où, quand Q représente $-COOR_4$, l'un des radicaux $R_2$ et $R_3$ peut être substitué par un groupe $-COOR_4$, ou bien $R_2$ ou $R_3$ peuvent être liés au radical $-C_nH_{2n+1-k}$ de telle sorte qu'il se forme un radical cycloalkylène en $C_{5-12}$, substitué par le ou les groupes $-(COOR_4)_k$ avec des radicaux $R_4$ identiques ou différents,

$R_4$ est l'hydrogène, un radical alkyle en $C_{1-20}$ pouvant être interrompu par 1 à 5 atomes de

carbone et substitué par un groupe $-OR_6$; alcényle en $C_{3-20}$, cycloalkyle en $C_{3-12}$, aryle en $C_{6-10}$ pouvant être substitué par un groupe alkyle en $C_{1-4}$; aralkyle en $C_{7-13}$, un hétérocycle oxygéné, à cinq ou six chaînons, pouvant être substitué par un ou deux groupes alkyle en $C_{1-4}$, ou le radical méthyle, substitué par un hétérocycle contenant un atome d'oxygène, à cinq ou six chaînons, non substitué, ou substitué par un ou deux groupes alkyle en $C_{1-4}$,

$R_5$ est l'hydrogène ou un radical alkyle en $C_{1-20}$, ou bien $R_4$ et $R_5$ forment avec l'atome d'azote auquel ils sont liés un hétérocycle à cinq ou six chaînons, pouvant être substitué par un ou deux groupes alkyle en $C_{1-4}$,

$R_6$ est un radical cycloalkyle en $C_{3-12}$, alcényle en $C_{3-20}$, aryle en $C_{6-10}$ pouvant être substitué par un ou deux groupes alkyle en $C_{1-4}$, ou bien aralkyle en $C_{7-13}$, et

$R_7$ est l'hydrogène, un radical alkyle en $C_{1-20}$, alcényle en $C_{3-20}$, cycloalkyle en $C_{3-12}$, aralkyle en $C_{7-13}$, ou aryle en $C_{6-10}$ pouvant être substitué par un ou deux groupes alkyle en $C_{1-4}$,

$R_8$ est l'hydrogène ou un radical alkyle en $C_{1-4}$, et

$R_9$ est l'hydrogène, un radical alkyle en $C_{1-4}$ ou un groupe $-COR_7$ dans lequel $R_7$ a la signification donnée ci-dessus, ou bien $R_8$ et $R_9$, avec l'atome d'azote auquel ils sont liés, forment un noyau à cinq ou six chaînons, non-substitué ou substitué par un ou deux groupes alkyle en $C_{1-4}$, et

$R_{10}$ et $R_{11}$, pour x = 1, indépendamment l'un de l'autre, sont chacun l'hydrogène, un radical alkyle en $C_{1-20}$, ou forment ensemble un radical alkylène en $C_{2-3}$ pouvant être substitué par 1–4 groupes alkyle en $C_{1-20}$, et, pour x = 0, $R_{10}$ est l'hydrogène ou un radical alkyle en $C_{1-20}$ et $R_{11}$ est un radical alkyle en $C_{1-5}$ à chaîne droite,

$R_{12}$ est un radical $-OH$, $-Cl$ ou $-N(R_5)(R_7)$, et

$R_{13}$ est un radical $-H$, alkyle en $C_{1-20}$ ou halogène, $R_5$ et $R_7$ ayant les significations données ci-dessus, à la condition que $R_1$ représente un radical de formule II quand $R_{12}$ est le radical $-OH$,

ainsi que des sels de composés de formule I avec des acides et bases organiques ou inorganiques.

2. Benzoquinones de formule I selon la revendication 1, dans laquelle les groupes R et $R_1$ se trouvent sur les positions 2 et 5 de la 1,4-benzoquinone.

3. Benzoquinones de formule I selon la revendication 1, dans laquelle $R_1$ est un radical de formule III

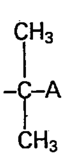

(III)

dans laquelle A est un radical alkyle ayant de 1 à 5 atomes de carbone.

4. Benzoquinones de formule I selon la revendication 1, dans laquelle R est un radical de formule II, dans laquelle Q est un radical $-COOR_4$, $-OCOR_7$ ou $-CN$, $R_1$ à $R_4$, $R_7$, p, q, k et n ayant les significations données dans la revendication 1.

5. Benzoquinones selon la revendication 4, caractérisées en ce que, dans le composé de formule I, R et $R_1$ sont chacun un radical de formule II dans laquelle Q est un radical $-COOR_4$, où $R_4$ est un radical alkyle en $C_{1-12}$, lequel est non-substitué ou substitué par un radical cycloalkyloxy en $C_{3-12}$ ou aryloxy en $C_{6-10}$, cycloalkyle en $C_{3-12}$, aryle en $C_{6-10}$, aralkyle en $C_{7-13}$ ou le radical méthyle, lequel est substitué par un noyau hétérocyclique non-substitué, à cinq ou six chaînons, contenant un atome d'oxygène, ou bien Q est le radical $-OCOR_7$, où $R_7$ est l'hydrogène, un radical alkyle en $C_{1-20}$, cycloalkyle en $C_{3-12}$, aralkyle en $C_{7-13}$ ou aryle en $C_{6-10}$ non-substitué, $R_2$ et $R_3$, indépendamment l'un de l'autre, sont chacun un radical alkyle ayant de 1 à 5 atomes de carbone, p et q valent chacun 1, et k et n ont les significations données dans la revendication 4.

6. Benzoquinones selon la revendication 5, caractérisées en ce qu'elles correspondent à la formule IV

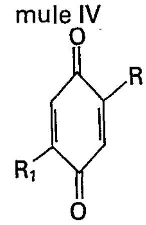

(IV),

dans laquelle R et $R_1$ sont chacun un radical de formule II, dans laquelle Q est le radical $-COOR_4$, $R_4$ étant un radical alkyle ayant de 1 à 8 atomes de carbone, ou cycloalkyle ayant de 5 à 8 atomes de carbone, ou bien représente $-OCOR_7$, où $R_7$ est l'hydrogène ou un radical alkyle ayant de 1 à 4 atomes de carbone, $R_2$ et $R_3$ sont chacun le radical méthyle, k vaut 1 et n vaut 1 à 10.

7. Benzoquinones de formule IV selon la revendication 6, dans laquelle n est le nombre 3, $R_2$ et $R_3$ sont chacun le radical méthyle et Q est le radical $-COOR_4$, $R_4$ étant un radical alkyle ayant de 1 à 8 atomes de carbone.

8. Benzoquinones de formule I selon la revendication 1, dans laquelle Q est un radical $-COOR_4$, $-CON(R_4)(R_5)$, $-OR_5$, $-OCOR_7$, $-N(R_8)(R_9)$, $-OPO(OR_{10})([0]_xR_{11})$, $-SO_2R_{12}$ ou $-CN$, k, p, q, n, R, $R_1$ à $R_3$, $R_5$ à $R_{12}$ et x ayant les significations données dans la revendication 1, et $R_4$ étant $-H$, un radical alkyle en $C_{1-20}$ pouvant être interrompu par 1–5 atomes d'oxygène et substitué par un groupe $-OR_6$, dans lequel $R_6$ a les significations données ci-dessus, et de plus $R_4$ représente un radical alcényle en $C_{3-20}$, cycloalkyle en $C_{3-12}$, aryle en $C_{6-10}$ pouvant être substitué par un groupe alkyle en $C_{1-4}$, aralkyle en $C_{7-13}$, un hétérocycle à cinq ou six chaînons, contenant de l'oxygène et pouvant être substitué par un ou deux groupes alkyle en $C_{1-4}$, ou le radical méthyle, lequel est substitué par un hétérocycle substitué, à cinq ou six chaînons, contenant un atome d'oxygène, ou substitué par un ou deux groupes alkyle en $C_{1-4}$.